(19) Europäisches Patentamt
European Patent Office
Office européen des brevets

(11) Numéro de publication: **0 037 344**
**B1**

(12) # FASCICULE DE BREVET EUROPEEN

(45) Date de publication du fascicule du brevet: **11.07.84**

(21) Numéro de dépôt: **81400520.3**

(22) Date de dépôt: **01.04.81**

(51) Int. Cl.³: **C 07 D 231/20**, C 07 D 403/12, C 07 D 401/12, C 07 D 413/12, A 61 K 31/415, A 61 K 31/445, A 61 K 31/535

(54) **Amino-alcoxy pyrazoles, procédé pour leur préparation, et médicaments les contenant.**

(30) Priorité: **01.04.80 FR 8007301**

(43) Date de publication de la demande:
**07.10.81 Bulletin 81/40**

(45) Mention de la délivrance du brevet:
**11.07.84 Bulletin 84/28**

(84) Etats contractants désignés:
**AT BE CH DE FR GB IT LI LU NL SE**

(56) Documents cités:
**FR - A - 2 167 997**

(73) Titulaire: **ETABLISSEMENTS NATIVELLE S.A.**
**27, rue de la Procession**
**F-75015 Paris (FR)**

(72) Inventeur: **Jarreau, François-Xavier**
**5, rue Louis Hervé**
**F-78000 Versailles (FR)**
Inventeur: **Koenig, Jean-Jacques**
**31, rue du Panorama**
**F-77670 Vernou-la-Celle s/Seine (FR)**

(74) Mandataire: **L'Helgoualch, Jean et al,**
**OFFICE PICARD 134 Boulevard de Clichy**
**F-75018 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 037 344 B1

Courier Press, Leamington Spa, England.

**Description**

La présente invention, réalisée dans les laboratoires du CERES (Centre Européen de Recherche Scientifique) concerne de nouveaux dérivés du pyrazole, et plus particulièrement des aminoalcoxy pyrazoles, un procédé pour leur préparation, et leur application en thérapeutique.

Des dérivés du pyrazole et de la pyrazolone sont décrits notamment dans le brevet français 2.167.997, au nom de la demanderesse, qui concerne en particulier des composés du type amino-1-pyrazole dont l'atome de carbone en position 5 est substitué par un groupe hydroxy ou alcoxy inférieur, présentant des propiétiés intéressantes permettant leur application à titre de médicaments.

La présente invention concerne des dérivés des composés précités, comportant un groupe alcoxy fonctionnel substitué sur l'atome de carbone en position 5 du noyau pyrazolique, leur conférant des propriétés spécifiques.

L'invention a pour objet des dérivés du type amino-alcoxy pyrazole représentés par la formule générale I:

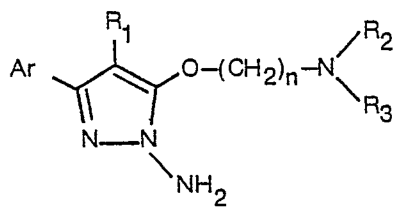

(I)

dans laquelle Ar représente un groupe phényle, benzyle, naphtyle, ou un groupe phényle substitué par un ou plusieurs atomes d'halogène, ou par un ou plusieurs groupes méthyle, cyano, nitro, hydroxy ou méthoxy; $R_1$ représente un atome d'hydrogène ou un groupe alkyle inférieur de 1 à 4 atomes de carbone; $R_2$ et $R_3$, identiques ou différents, représentent un groupe alkyle inférieur de 1 à 3 atomes de carbone, ou forment avec l'atome d'azote auquel ils sont rattachés, un hétérocycle de 5 à 7 chainons, pouvant comporter un deuxième hétéroatome choisi parmi l'azote et l'oxygène, cet hétérocycle pouvant être substitué, le cas échéant par un groupe phényle; n est un entier de 1 à 4; ainsi que leurs sels avec un acide minéral ou organique.

L'invention a également pour objet l'application des dérivés de formule générale I à titre de médicaments, notamment pour le traitement des états dépressifs, des troubles polyfonctionnels, des migraines et des affections cardiovasculaires.

Dans la formule générale I représentée ci-dessus, Ar peut être un groupe phényle, ou un groupe benzyle, 1-naphtyle, 2-naphtyle, ou un group phényle substitué, et par exemple un groupe o-, m- ou p-chlorophényle, di chloro-2,4 phényle, trichloro-2,4,6 phényle, p-hydroxyphényle, p-méthoxyphényle, p-cyanophényle, p-nitrophényle, ou tolyle. $R_1$ est un atome d'hydrogène ou un groupe alkyle tel que méthyle, éthyle, n-propyle, isopropyle, ou butyle, $R_2$ et $R_3$, identiques ou différents, représentent un groupe alkyle tel que méthyle, éthyle, n-propyle, ou isopropyle; lorsque $R_2$ et $R_3$ forment un hétérocycle de 5 à 7 chainons avec l'atome d'azote auquel ils sont rattachés, ce groupe hétérocyclique peut être un groupe pyrrolyle, azépinyle, pyrrolidinyl, pyrrolinyle ou pipéridyle; ce groupe hétérocyclique peut contenir un deuxième hétéroatome choisi parmi l'azote ou l'oxygène, et être par exemple un groupe imidazolyle, pyrazolyle, pyrazinyle, pyrimidinyle, pyrazinyle, oxazolyle, imidazolinyle, pyrazolidinyle, pyrazolinyle, pipérazinyle, phényl-4 pipérazinyle, ou morpholinyle.

Parmi les dérivés de formule générale I, l'invention concerne de préférence ceux pour lesquels Ar est un groupe phényle ou un groupe phényle substitué par un ou plusieurs atomes de chlore, et plus particulièrement un groupe p-chlorophényle, $R_1$ est un atome d'hydrogène ou un groupe alkyle inférieur, $R_2$ et $R_3$ représentent un atome d'hydrogène ou un groupe alkyle inférieur, ou forment ensemble avec l'atome d'azote auquel ils sont rattachés, un groupe pipérazinyle, phényl-4 pipérazinyle, pipéridyle, pyrrolidinyle, ou morpholinyle, et n est 2 ou 3.

L'invention concerne également les sels des dérivés de formule générale I, et en particulier les sels pharmaceutiquement acceptables, obtenus par action des acides usuels minéraux ou organiques tels que les acides chlorhydrique, sulfurique, phosphorique, oxalique, lactique, citrique, etc. Ces sels sont obtenus selon les procédés usuels de la technique.

Les aminoalcoxy-pyrazoles suivant l'invention, représentés par la formule générale I ci-dessus, peuvent être préparés à partir des aminopyrazolones correspondantes représentées par la formule générale II:

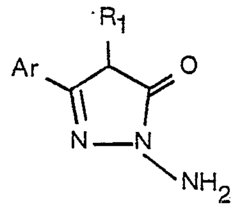

(II)

dans laquelle Ar et $R_1$ conservent les définitions indiquées ci-dessus, par réaction avec une haloalkylamine de formule générale III:

$$X—(CH_2)_n—NR_2R_3$$

dans laquelle X représente un atome d'halogène, n, $R_2$ et $R_3$ conservent les définitions indiquées dans la formule générale I, en présence d'un hydrure, un amidure ou un alcoolate de métal alcalin dans un solvant organique.

Les aminopyrazolones de formule générale II peuvent être préparées conformément au procédé décrit dans le brevet français 2.167.997, par transposition interne sur un dérivé d'hydrazino-5 isoxazole, en présence d'hydrazine anhydre en excès.

Les haloalkylamines utilisées comme réactifs conformément au procédé suivant l'invention sont généralement disponibles dans le commerce sous forme de chlorhydrates. Il est avantageux de transformer ces chlorhydrates en bases correspondantes, au moment de leur utilisation, par dissolution dans une solution saturée de carbonate de potassium et extraction à l'éther, suivant la méthode décrite dans Fieser Reagents IV, 267.

Comme exemples d'haloalkylamines on peut citer en particulier les chloroalkylamines telles que la N-(chloro-2 éthyl)-diméthylamine, la N-(chloro-3 propyl)-diméthylamine, ou la N-(chloro-2 éthyl)diisopropylamine, ou un hétérocycle N-chloroalkylé tel qu'un cycle de 5 à 7 chainons comportant le cas échéant un deuxième hétéroatome tel que l'azote ou l'oxygène, substitué sur l'atome d'azote par un groupe chloroalkyle, et de préférence la N-(chloro-2 éthyl)-morpholine, la N-(chloro-2 éthyl)-pyrrolidine, la N-(chloro-3 propyl)-pipéridine, ou la N-(chloro-2 éthyl) phényl-4-pipérazine.

L'aminopyrazolone de départ, représentée par la formule générale II, est de préférence une amino-1 pyrazol-5-one telle que l'amino-1 p-chlorophényl-3 pyrazolone, l'amino-1 phényl-3 pyrazolone, ou l'amino-1 phényl-3 méthyl-4 pyrazolone.

Conformément au procédé suivant l'invention, la réaction s'effectue avantageusement dans un solvant organique contenant l'amino pyrazolone de départ, en présence d'un hydrure ou d'un amidure de métal alcalin aquel on ajoute progressivement un chloroalkylamine conforme à la formule générale III. On peut utiliser par exemple l'hydrure de sodium ou de potassium, ou l'amidure de sodium.

La réaction s'effectue à température ambiante, mais il peut être avantageux de porter le milieu réactionnel à une température comprise entre 20 et 100°C, et de préférence entre 50 et 80°C.

La réaction s'effectue dans un solvant organique approprié, et par exemple un solvant aprotique tel que le tétrahydrofuranne, le diméthylformamide, le diméthylsulfoxyde, le dioxanne, le diglyme, un éther d'alkyle, etc.; lorsque la base est un alcoolate de métal alcalin, il est avantageux d'utiliser comme solvant l'alcool correspondant, par exemple le méthanolate de sodium dans le méthanol.

Les exemples indiqués ci-après illustrent plus en détail l'invention sans en limiter la portée.

Exemple 1

amino-1 p-chlorophényl-3 morpholinoéthoxy-5 pyrazole.

Dans un tricol de 1 l. muni d'un réfrigérant et d'un tube à chlorure de calcium, d'une arrivée d'azote, d'une ampoule de coulée et d'un agitateur, on place 3,05 g d'amino-1 p-chloro phényl-3 pyrazolone dans 200 ml de diméthylformamide distillé. On fait barboter de l'azote dans la solution pendant 15 mn, puis on refroidit à environ 0—5°C sur bain de glace. On ajoute 0,4 g d'hydrure de sodium et on maintient sous agitation 15 mn en laissant la température revenir à températur ambiante.

On refroidit à nouveau à 0—5°C sur bain de glace, et on ajoute goutte à goutte, sous agitation, 4,5 g de chloro-2 N-éthylmorpholine. On laisse réagir à température ambiante, en suivant l'évolution de la réaction par chromatographie sur couche mince.

On refroidit à nouveau à 0—5°C sur bain de glace, et on ajoute goutte à goutte, sous agitation, 4,5 g de chloro-2 N-éthylmorpholine. On laisse réagir à température ambiante, en suivant l'évolution de la réaction par chromatographie sur couche mince.

Quand la réaction est terminée, on élimine le diméthylformamide par distillation sous pression réduite (0,1 mmHg environ), on reprend le résidu avec 300 ml d'eau distillée et après lavage au chloroforme, on extrait les phases organiques avec un solution à 10% d'acide citrique. On alcalinise par du carbonate de sodium et on extrait avec de l'éther éthylique. Après lavage par une solution aqueuse saturée de chlorure de. sodium, séchage et évaporation sous vide, on obtient 3,10 g d'amino-1 p-chloro-phényl-3 morpholinoéthoxy-5 pyrazole, sous forme de cristaux incolores.

Point de fusion F = 120—121°C

Chromatographie sur couche mince (CCM): Rf = 0,50 (acétate d'éthyl + diéthylamine (10%)

Spectre de RMN: $\delta$ = 2,5 (m, 4 H) 2,7 (t, 2 H) 3,7 (m, 4 H) 4,2 (t, 2 H) 5,2 (s, 2 H mobiles) 5,7 (s, 1 H) 7,4 (q, 4 H) ppm ($CDCl_3$)

Exemple 2

Amino-1 p-chlorophényl-3 pipéridino éthoxy-5 pyrazole

On procédé comme dans l'exemple 1 à partir de la même pyrazolone de départ, mais en remplaçant la chloroéthylmorpholine par de la chloroéthylpipéridine que l'on ajoute goutte à goutte, et en

effectuant la réaction à une température de 60°C. On obtient ainsi l'amino-1 p-chlorophényl-3 pipéridinoéthoxy-5 pyrazole en 3 heures; après purification comme indiqué dans l'exemple 1, le rendement est de 55%.

Point de fusion F=119°C
CCM Rf = 0,70 (acétate d'éthyle + diéthylamine 10%)
Spectre de RM : $\delta$ = 1,5 (6 H) 2,4 (4 H) 2,7 (t, 2 H) 4,2 (t 2 H) 5,3 (2 H mobiles) 5,7 (s, 1 H) 7,4 (q, 4 H) ppm. ($CDCl_3$)

Exemple 3

Amino-1 p-chlorophényl-3 pipéridinopropoxy-5 pyrazole

On opère comme dans l'exemple précédent en utilisant la chloropropylpipéridine à la place de la chloro-éthylpipéridine. Aprés extraction à l'acide citrique puis alcalinisation au carbonate de sodium comme indiqué dans l'exemple 1, on extrait l'amino-1 p-chlorophényl-3 pipéridino-propoxy-5 pyrazole de la phase aqueuse par de l'hexane.

Point de fusion F=90°C
CCM Rf = 0,63 (acétate d'éthyle + diéthylamine 10%)
Spectre de RMN: $\delta$ = 1,4 (m, 8 H) 2,2 (m, 6 H) 4,0 (t, 2 H) 5,1 (s, 2 H mobiles) 5,5 (s, 1 H) 7,3 (q, 4 H) ppm. ($CDCl_3$)

Exemple 4

Amino-1 p-chlorophényl-3 pyrrolidinoéthoxy-5 pyrazole

On procède comme dans l'exemple 1, également à partir de l'amino-1 p-chlorophényl-3 pyrazolone, mais en remplaçant la chloroéthylmorpholine par de la chloroéthylpyrrolidine.

Aprés purification et extraction de la phase aqueuse par de l'éther éthylique, on obtient l'amino-1 p-chlorophényl-3 pyrrolidinoéthoxy-5 pyrazole.

Point de fusion F=131°C
CCM Rf = 0,50 (acétate d'éthyle + diéthylamine 10%)
Spectre de RMN: $\delta$ = 1,8 (m, 4 H) 2,5 (m, 4 H) 2,9 (t, 2 H) 4,2 (t, 2 H) 5,1 (2 H mobiles) 5,7 (s, 1H) 7,4 (q, 4 H) ppm. ($CDCl_3$)

Exemple 5

Amino-1 p-chlorophényl-3 (phényl-4 pipérazino)-éthoxy-5 pyrazole

On dissout 3 g d'amino-1 p-chlorophényl-3 pyrazolone dans 200 ml de tétrahydrofuranne. On fait barboter de l'azote dans cette solution puis on refroidit au bain de glace et on ajoute 0,45 g d'hydrure de potassium. On ajoute ensuite progressivement 6,2 g de (chloro-2 éthyl)-1 phényl-4 pipérazine en maintenant le mélange réactionnel sous agitation.

Quand la réaction est terminée, on élimine le tétrahydrofuranne par distillation sous pression réduite, on lave et on purifie suivant la technique indiquée dans l'exemple 1. L'extraction de la phase aqueus alcaline se fait par l'acétate d'éthyle.

Après purification on recueille 3,25 g d'amino-1 p-chlorophényl-3 (phényl-4 pipérazino)-éthoxy-5 pyrazole.

Point de fusion F=174°C
CCM Rf = 0,57 (acétate d'éthyle + diéthylamine 10%)
Spectre de RMN: $\delta$ = 2,8 (m, 6 H) 3,2 (m, 4 H) 4,3 (t, 2 H) 4,4 (2 H mobiles) 5,8 (2, 1 H) 6,8 à 7,7 (m, 9 H) ppm ($CDCl_3$)

Exemple 6

Amino-1 phényl-3 pyrrolidinoéthoxy-5 pyrazole

On procède comme dans l'exemple 1 en remplaçant l'amino-1 p-chlorophényl-3 pyrazolone par l'amino-1 phényl-3 pyrazolone, dans le diméthylformamide, sur laquelle on fait agir goutte à goutte la chloroéthyl-pyrrolidine en présence d'hydrure de sodium.

Après purification suivant la technique indiquée dans l'exemple 1, on obtient l'amino-1 phényl-3 pyrrolidinoéthoxy-5 pyrazole.

Point de fusion F=138°C
CCM Rf = 0,70 (acétate d'éthyle + diéthylamine 10%)
Spectre de RMN: $\delta$ = 1,8 (m, 4 H) 2,6 (m, 6 H) 2,7 (t, 2 H) 4,2 (t, 2 H) 5,1 (2 H mobiles) 5,7 (s, 1 H) 7,1 à 7,7 (2 m, 5 H) ppm. ($CDCl_3$)

Exemple 7

Amino-1 phényl-3 morpholinoéthoxy-5 pyrazole

On procède comme dans l'exemple 1 en utilisant comme produit de départ l'amino-1 phényl-3 pyrazolone au lieu de l'amino-1 p-chlorophényl-3 pyrazolone.

On obtient ainsi l'amino-1 phényl-3 morpholino-éthoxy-5 pyrazole.

Point de fusion F=139°C

CCM Rf = 0,75 (acétate d'éthyle + diéthylamine 10%)

Spectre de RMN: $\delta$ = 2,5 (m, 4 H) 2,8 (t, 2 H) 3,8 (m, 4 H) 4,2 (t, 2 H) 5,2 (2 H mobiles) 5,7 (s, 1 H) 7,1 à 7,8 (2 m, 5 H) ppm. ($CDCl_3$)

Exemple 8

Amino-1 phényl-3 méthyl-4 morpholinoéthoxy-5 pyrazole

On procède comme dans l'exemple 1 en utilisant comme produit de départ l'amino-1 phényl-3 méthyl-4 pyrazolone au lieu de l'amino-1 p-chloro-phényl-3 pyrazolone.

On obtient ainsi l'amino-1 phényl-3 méthyl-4 morpholinoéthoxy-5 pyrazole.

Point de fusion F=96°C

CCM Rf = 0,70 (acétate d'éthyle + diéthylamine 10%)

Spectre de RMN: $\delta$ = 2,1 (s, 3 H) 2,5 (m, 4 H) 2,6 (t, 2 H) 3,6 (m, 4 H) 4,2 (t, 2 H) 5,4 (2 H mobiles) 7,1 à 7,8 (2 m, 5 H) ppm ($CDCl_3$)

Exemple 9

Amino-1 phényl-3 méthyl-4 pyrrolidinoéthoxy-5 pyrazole

On procède comme dans l'exemple précédent, en faisant agir la chloroéthyl-pyrrolidine goutte à goutte sur l'amino-1 phényl-3 méthyl-4 pyrazolone, en présence d'amidure de sodium dans le tétrahydrofuranne.

On obtient ainsi l'amino-1 phényl-3 méthyl-4 pyrrolidinoéthoxy-5 pyrazole.

Point de fusion F=109°C

CCM Rf = 0,70 (acétate d'éthyle + diéthylamine 10%)

Spectre de RMN: $\delta$ = 1,7 (m, 4 H) 2,1 (s, 3 H) 2,5 (m, 4 H) 2,7 (t, 2 H) 4,2 (t, 2 H) 5,3 (2 H mobiles) 7,1 à 7,8 (2 m, 5 H) ppm ($CDCl_3$)

Exemple 10

Amino-1 phényl-3 diméthylaminoéthoxy-5 pyrazole

On procède comme dans l'exemple 1, en faisant agir la N-chloroéthyldiméthylamine goutte à goutte sur l'amino-1 phényl-3 pyrazolone, dans le diméthylformamide, en présence d'hydrure de sodium.

On obtient ainsi l'amino-1 phényl-3 diméthylaminoéthoxy-5 pyrazole.

Point de fusion F=80°C

CCM Rf = 0,75 (acétate d'éthyle + diéthylamine 10%)

Spectre de RMN: $\delta$ = 2,2 (s, 6 H) 2,6 (t, 2 H) 4,0 (t, 2 H) 5,3 (2 H mobiles) 5,6 (s, 1 H) 7,1 à 7,7 (2m, 5 H) ppm ($CDCl_3$)

Les expérimentations effectuées sur les amino alcoxy pyrazoles de formule générale I conformes à la présente invention ont mis en évidence d'intéressantes propriétés pharmacologiques permettant d'envisager leur application en thérapeutique.

Les essais toxicologiques ont permis de déterminer, pour les dérivés de formule générale I décrits dans les exemples 1 à 10 ci-dessus, une dose léthale DL50 comprise généralement entre 500 et 1000 mg/kg.

Les aminoalcoxypyrazoles suivant l'invention possèdent une activité anti-dépressive comparable à celle des antidépresseurs tricycliques et de l'amphétamine, mais s'exerçant différemment. Cette activité a été vérifiée au niveau des stéréotypies induites par le 5-hydroxytryptophane, précurseur de la sérotonine. Sur ce test on constate que les aminoalcoxy-pyrazoles augmentent les stéréotypies, mais s'opposent à l'hypothermie.

Cette activité antidépressive se trouve confirmée par la vérification d'une activité anticataleptique pratiquement totale au niveau de la catalepsie induite par un neuroleptique (la chlorpromazine).

Les tests des convulsions induites par le pentatétrazole et de l'électrochoc font apparaitre une activité anticonvulsivante trés nette.

Enfin, les aminoalcoxypyrazoles suivant l'invention présentent une activité cardiovasculaire se manifestant par une forte activité déprimante cardiaque, un effet de ralentissement de la fréquence cardiaque sans diminution de la pression ni de la force des contractions, des propriétés antiarythimiques et une importante action spasmolytique.

Ces propriétés montrent que les aminoalcoxypyrazoles de formule générale I conformes à l'invention peuvent être utilisés en médecine humaine ou vétérinaire, notamment pour le traitement des états dépressifs et des affections cardiques.

Les aminoalcoxypyrazoles suivant l'invention peuvent être administrés sous les formes usuelles de la technique, par exemple sous forme de comprimés, gélules, capsules, dragées, suppositoires, solutés injectables ou sirops, le principe actif étant dilué dans un support pharmaceutiquement acceptable convenablement choisi.

La posologie peut varier en fonction du sujet traité et de l'affection en cause. Les doses administrées journallement sont généralement comprises entre 5 et 200 mg pour l'administration par voie orale chez l'homme.

**Revendications**

1. Aminoalcoxypyrazoles de formule générale (I):

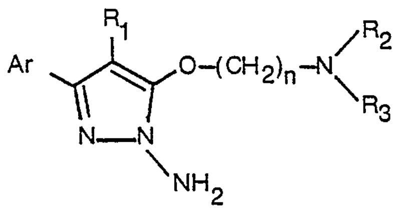

(I)

dans laquelle Ar représente un groupe phényle, benzyle, naphtyle, ou un groupe phényle substitué par un ou plusieurs atomes d'halogène, ou par un ou plusieurs groupes méthyle, cyano, nitro, hydroxy ou méthoxy; $R_1$ représente un atome d'hydrogène ou un groupe alkyle inférieur de 1 à 4 atomes de carbone; $R_2$ et $R_3$, identiques ou différents, représentent un groupe alkyle inférieur de 1 à 3 atomes de carbone, ou forment avec l'atome d'azote auquel ils sont rattachés, un hétérocycle de 5 à 7 chainons, pouvant comporter un deuxième hétéroatome choisi parmi l'azote et l'oxygène, cet hétérocycle pouvant être substitué le cas échéant par un groupe phényle; n est un entier de 1 à 4; ainsi que leurs sels avec un acide minéral ou organique.

2. Aminoalcoxypyrazoles selon la revendication 1, caractérisés en ce que Ar est un groupe phényle, ou un groupe phényle substitué par un ou plusieurs atomes d'halogène.

3. Aminoalcoxypyrazoles selon la revendication 2, caractérisés en ce que Ar est un groupe phényle ou p-chlorophényle.

4. Aminoalcoxypyrazoles selon l'une quelconque des revendications 1 à 3, caractérisés en ce que $R_2$ et $R_3$ représentent un groupe méthyle, ou forment avec l'atome d'azote auquel ils sont rattachés, un groupe pyrrolyle, pyrrolidinyle, pipéridyle, pipéridinyle, morpholinyle ou pipérazinyle, ces groupes pouvant être substitués le cas échéant par un group phényle.

5. Aminoalcoxypyrazoles selon la revendication 1, caractérisés en ce qu'ils sont choisis parmi:
l'amino-1 p-chlorophényl-3 morpholinoéthoxy-5-pyrazole,
l'amino-1 p-chlorophényl-3 pipéridinoéthoxy-5-pyrazole,
l'amino-1 p-chlorophényl-3 pipéridinopropoxy-5-pyrazole,
l'amino-1 p-chlorophényl-3 pyrrolidinoéthoxy-5-pyrazole,
l'amino-1 p-chlorophényl-3 (phényl-4 pipérazino)-éthoxy-5 pyrazole.

6. Aminoalcoxypyrazoles selon la revendication 1, caractérisés en ce qu'ils sont choisis parmi:
l'amino-1 phényl-3 pyrrolidinoéthoxy-5 pyrazole,
l'amino-1 phényl-3 morpholinoéthoxy-5 pyrazole,
l'amino-1 phényl-3 méthyl-4 morpholinoéthoxy-5 pyrazole,
l'amino-1 phényl-3 méthyl-4 pyrrolidinoéthoxy-5 pyrazole,
l'amino-1 phényl-3 diméthylaminoéthoxy-5 pyrazole.

7. Procédé de préparation d'aminoalcoxypyrazoles selon la revendication 1, caractérisé en ce qu'on fait réagir une amino-1 pyrazolone de formule générale II:

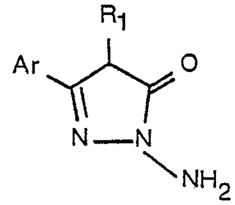

(II)

dans laquelle Ar et $R_1$ on la même signification que dans la revendication 1, avec une haloalkylamine de formule III:

$$X—(CH_2)_n—NR_2R_3 \qquad (III)$$

dans laquelle X représente un atome d'halogène, n, $R_2$ et $R_3$ conservent les définitions indiquées dans la revendication 1, en présence d'un hydrure, un amidure ou un alcoolate de métal alcalin dans un solvant organique.

8. Procédé selon la revendication 7, caractérisé en ce que X représente un atome de chlore.

9. Procédé selon la revendication 7, caractérisé en ce que la réaction s'effectue en présence d'hydrure de sodium, d'hydrure de potassium, ou d'amidure de sodium.

10. Procédé selon la revendication 7, caractérisé en ce que l'amino-1 pyrazolone de formule (II) est l'amino-1 phényl-3 pyrazolone, l'amino-1 phényl-3 méthyl-4 pyrazolone ou l'amino-1 p-chlorophényl-3 pyrazolone.

11. Médicament caractérisé en ce qu'il est constitué par un aminoalcoxypyrazole selon l'une quelconque des revendictions 1 à 6.

12. Composition pharmaceutique, caractérisée en ce qu'elle contient à titre de principe actif un médicament selon la revendication 11, associé à un ou plusieurs excipients.

**Claims**

1. Aminoalkoxypyrazoles of the general formula (I)

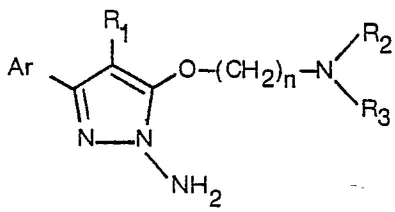

(I)

wherein Ar is a phenyl, benzyl or naphtyl group, or a phenyl group substituted by one or more halogen atoms, or by one or more alkyl, cyano, nitro, hydroxy or methoxy groups; $R_1$ is a hydrogen atom or a lower alkyl group from 1 to 4 carbon atoms; $R_2$ and $R_3$, which may be the same or different, are a lower alkyl group from 1 to 3 carbon atoms, or form, with the nitrogen atom to which they are attached a 5- to 7-membered heterocyclic ring which can further comprise a second heteroatom selected from nitrogen and oxygen, and said heterocyclic ring is possibly substituted by a phenyl group; n is an integer or from 1 to 4; as well as the mineral or organic acid salts thereof.

2. Aminoalkoxypyrazoles according to claim l, characterized in that Ar is a phenyl group, or a phenyl group substituted by one or more halogen atoms.

3. Aminoalkoxypyrazoles according to claim 2, characterized in that Ar is a phenyl group or a p-chlorophenyl group.

4. Aminoalkoxypyrazoles according to any of claims 1 to 3, characterized in that $R_2$ and $R_3$ are a methyl group, or form, with the nitrogen atom to which they are attached, a pyrrolyl, pyrrolidinyl, piperidyl, piperidinyl, morpholinyl or piperazinyl group, said groups being unsubstituted or substituted by a phenyl group.

5. Aminoalkoxypyrazoles according to claim 1, selected from the group consisting of
1-amino-3-(p-chlorophenyl)-5-(morpholinoethoxy)-pyrazole,
1-amino-3-(p-chlorophenyl)-5-(piperidinoethoxy)-pyrazole,
1-amino-3-(p-chlorophenyl)-5-(piperidinopropoxy)-pyrazole,
1-amino-3-(p-chlorophenyl)-5-(pyrrolidinoethoxy)-pyrazole,
1-amino-3-(p-chlorophenyl)-5-[(4-phenylpiperazino)ethoxy]-pyrazole.

6. Aminoalkoxypyrazoles according to claim 1, selected from the group consisting of:
1-amino-3-phenyl-5-(pyrrolidinoethoxy)-pyrazole,
1-amino-3-phenyl-5-(morpholinoethoxy)-pyrazole,
1-amino-3-phenyl-4-methyl-5-(morpholinoethoxy)-pyrazole,
1-amino-3-phenyl-4-methyl-5(pyrrolidinoethoxy)-pyrazole,
1-amino-3-phenyl-5-[(dimethylamino)ethoxy]-pyrazole.

7. A process for the preparation of the aminoalkylpyrazoles of claim 1, characterized in that an amino-1 pyrazolone of the general formula (II):

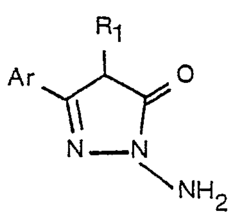

(II)

wherein Ar and $R_1$ have the same definition as in claim 1, is caused to react with haloalkylamine of the general formula (III):

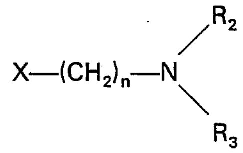

(III)

wherein X is a halogen atom, n, $R_2$ and $R_3$ have the same definitions as in claim 1, in the presence of a hydride, an amide or an alkali metal alcoholate in an organic solvent.

8. The process of claim 7, characterized in that X is a chlorine atom.

9. The process of claim 7, characterized in that the reacting is in the presence of sodium hydride, potassium hydride or sodium amide.

10. The process of claim 7, characterized in that the 1-aminopyrazolone of the formula (II) is 1-amino-3-phenylpyrazolone, 1-amino-3-phenyl-4-methylpyrazolone or 1-amino-3-(p-chlorophenyl)-pyrazolone.

11. A drug characterized in that it consists of an aminoalkyloxypyrazole according to any of claims 1 to 6.

12. A pharmaceutical composition, characterized in that it comprises a drug according to claim 11 as active principle, along with one or more excipients.

**Patentansprüche**

1. Aminoalkoxypyrazole der allgemeinen Formel (I):

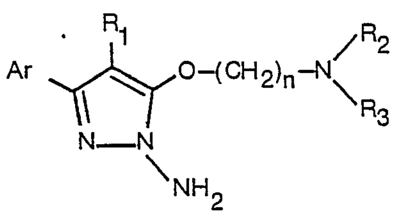

(I)

worin Ar eine Phenyl-, Benzyl- oder Naphthylgruppe oder eine durch ein oder mehrere Halogenatome oder durch eine oder mehrere Methyl-, Cyano, Nitro-, Hydroxy- oder Methoxygruppen substituierte Phenylgruppe darstellt, $R_1$ ein Wasserstoffatom oder eine niedere Alkylgruppe mit 1 bis 4 Kohlenstoffatomen bedeutet, die untereinander gleichen oder voneinander verschiedenen Reste $R_2$ und $R_3$ für eine niedere Alkylgruppe mit 1 bis 3 Kohlenstoffatomen stehen oder zusammen mit dem diese Reste tragenden Stickstoffatom einen 5- bis 7-gliedrigen heterocyclischen Ring bilden, welcher als zweites Heteroatom ein Stickstoffatom oder Sauerstoffatom aufweisen kann und gegebenenfalls durch eine Phenylgruppe substituiert ist, und n eine ganze Zahl von 1 bis 4 bedeutet, sowie ihre Salze mit einer Mineralsäure oder einer organischen Säure.

2. Aminoalkoxypyrazole nach Anspruch 1, dadurch gekennzeichnet, daß Ar eine Phenylgruppe oder eine durch ein oder mehrere Halogenatome substituierte Phenylgruppe bedeutet.

3. Aminoalkoxypyrazole nach Anspruch 2, dadurch gekennzeichnet, daß Ar eine Phenylgruppe oder eine p-Chlorphenylgruppe ist.

4. Aminoalkoxypyrazole nach irgendeinem der Ansprüche 1 bis 3, dadurch gekennzeichnet, daß $R_2$ und $R_3$ eine Methylgruppe darstellen oder zusammen mit dem diese Reste tragenden Stickstoffatom eine Pyrrolyl-, Pyrrolidinyl-, Piperidyl-, Piperidinyl-, Morpholinyl- oder Piperazinylgruppe darstellen, wobei diese Gruppen gegebenenfalls durch eine Phenylgruppe substituiert sind.

5. Aminoalkoxypyrazole nach Anspruch 1, dadurch gekennzeichnet, daß sie unter
1-Amino-3-(p-chlorphenyl)-5-morpholinoäthoxy-pyrazol,
1-Amino-3-(p-chlorphenyl)-5-piperidinoäthoxy-pyrazol,
1-Amino-3-(p-chlorphenyl)-5-piperidinopropoxy-pyrazol,
1-Amino-3-(p-chlorphenyl)-5-pyrrolidinoäthoxy-pyrazol und
1-Amino-3-(p-chlorphenyl)-5-[(4-phenyl-piperazino)-äthoxy]-pyrazol ausgewählt sind.

6. Aminoalkoxypyrazole nach Anspruch 1, dadurch gekennzeichnet, daß sie unter
1-Amino-3-phenyl-5-pyrrolidinoäthoxy-pyrazol,
1-Amino-3-phenyl-5-morpholinoäthoxy-pyrazol,
1-Amino-3-phenyl-5-(4-methyl-morpholinoäthoxy)-pyrazol,
1-Amino-3-phenyl-5-(4-methyl-pyrrolidinoäthoxy)-pyrazol und
1-Amino-3-phenyl-5-dimethylaminoäthoxy-pyrazol ausgewählt sind.

7. Verfahren zum Herstellen von Diaminoalkoxypyrazolen nach Anspruch 1, dadurch gekennzeichnet, daß ein 1-Amino-pyrazolon der allgemeinen Formel (II):

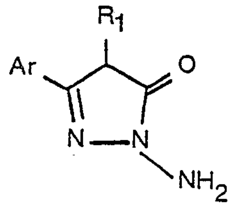

(II)

in welcher Ar und $R_1$ die in Anspruch 1 angegebene Bedeutung besitzen, mit einem Halogenalkylamin der allgemeinen Formel (III):

$$X—(CH_2)_n—NR_2R_3 \quad (III)$$

in welcher X ein Halogenatom darstellt und n, $R_2$ und $R_3$ die in Anspruch 1 angegebene Bedeutung besitzen, in Anwesenheit eines Hydrids, eines Amids oder eines Alkoholats eines Alkalimetalls in einem organischen Lösungsmittel umgesetzt wird.

8. Verfahren nach Anspruch 7, dadurch gekennzeichnet, daß X ein Chloratom darstellt.

9. Verfahren nach Anspruch 7, dadurch gekennzeichnet, daß die Umsetzung in Anwesenheit von Natriumhydrid, Kaliumhydrid oder Natriumamid vorgenommen wird.

10. Verfahren nach Anspruch 7, dadurch gekennzeichnet, daß das 1-Amino-pyrazolon der Formel (II) 1-Amino-3-phenyl-pyrazolon, 1-Amino-3-phenyl-4-methyl-pyrazolon oder 1-Amino-3-(p-chlorphenyl)-pyrazolon ist.

11. Heilmittel, dadurch gekennzeichnet, daß es von einem Aminoalkoxypyrazol gemäß irgendeinem der Ansprüche 1 bis 6 gebildet ist.

12. Pharmazeutische Mischung, dadurch gekennzeichnet, daß sie als Wirkstoff ein Heilmittel gemäß Anspruch 11 zusammen mit einem oder mehreren Exzipientia enthält.